# EUROPEAN PATENT SPECIFICATION

(11) **EP 2 020 901 B1**
(45) Date of publication and mention of the grant of the patent: **20.07.2016**
(21) Application number: 07795247.1
(22) Date of filing: 24.05.2007
(51) Int. Cl.: A61B 1/018

(54) **ENDOSCOPIC SLEEVE SEAL**
ENDOSKOPISCHE DICHTUNGSHÜLSE
ÉTANCHÉITÉ DE MANCHE ENDOSCOPIQUE

(30) Priority: 01.06.2006 US 810387 P
(43) Date of publication of application: 11.02.2009
(73) Proprietor: Cook Medical Technologies LLC, Bloomington, IN 47404 (US)
(72) Inventor: AYALA, Juan, Carlos, Santiago (CL); RUCKER, Brian, K., King, NC 27021 (US)
(74) Representative: Garratt, Peter Douglas
(86) International application number: PCT/US2007/012313
(87) International publication number: WO 2007/142844

(56) References cited:
- EP-A- 1 537 891
- WO-A-01/02044
- WO-A-2005/011791
- US-A- 5 167 636

## Description

### FIELD OF THE INVENTION

The invention relates to medical sealing devices, particularly those used in conjunction with endoscopes.

### BACKGROUND OF THE INVENTION

When an endoscope is used to view a passageway, such as the stomach, colon, intestine, bile duct, etc., a gas or liquid is often infused into the passageway to insufflate the passageway. Insufflating the passageway causes the passageway to expand, thus enabling the medical professional to better visualize and maneuver within the area in which the endoscopic procedure is being performed.

When a sufficient amount of fluid (including, but not limited to, gas, bile, blood, and radiopaque contrast fluid) is present in the passageway, back pressure occurs that may cause the fluid to leak out from the working channel of the endoscope; often times blood and bile puddle on the floor or on the person performing the procedure. In addition, surface tension also known as capillary- action can also cause fluid leakage even when no insufflation or backpressure is present. This is because the catheters and wire guides as well as the endoscope channels are small enough that they draw fluid out through the user end, even with no insufflation or backpressure. It is preferred that the fluid not leak from the endoscope while the endoscopic procedure is being performed, because such leakage may interfere with a successful outcome of the procedure and may contaminate the working area.

Seals are typically used to inhibit fluid leakage from the working channel of the endoscope. While traditional seals may seal around a single round- shaped device, most seals are incapable of inhibiting the escape of fluid from around one or more medical devices having a combined irregular cross-sectional profile that are disposed through the working channel of the endoscope. Furthermore, current seals often result in friction around the devices inserted therethrough. Friction inhibits the medical professional from using tactile feedback as a means for determining how the medical devices ought to be manipulated.

Reference is herein directed to WO 01/02044 in which an access member is adapted to extend from the outside of the body through the canal which may be an artificial canal extending from the user's abdominal wall to the bladder and into the bladder, and has at least one cavity extending substantially throughout the length of the access member. The walls of the access member are made from a flexible material. Reference is further directed to EP 1 537 891 which discloses an over tube formed by a tube body, and a gripping part in which a liquid storing part is formed. A connecting port formed at a tip end of the gripping part is fitted into a base end opening of the tube body in a watertight state, so that the gripping part is constructed attachably and detachably with respect to the tube body. The liquid storing part is formed into a spherical shape with a larger diameter than a diameter of the tube body, and an arc-shaped recessed portion is formed in an inside of the liquid storing part. A sponge formed into a donut shape is housed in this recessed portion. During an operation, a body fluid flowing backward from a gap between the tube body and an insertion section is stored in the recessed portion and is absorbed in the sponge, thus preventing liquid leakage from the recessed portion. Reference is also directed to US5 167 636 in there is provided a sealing assembly for sealing a cannula used in medical procedures. The cannula is configured to allow passage therethrough of medical devices. The cannula is attached to a conduit piece formed to define at least one channel. A compressible annular seal is positioned in the at least one channel to allow passage through its central aperture of a medical device. A mechanism is provided for compressing the compressible annular seal to provide an airtight seal across the at least one channel. A duckbill flap valve is also positioned in the at least one channel adjacent to the compressible annular seal to receive the medical device after its insertion through the central aperture of the compressible annular seal. Reference is further directed to WO 2005011791 in which there is provided a wire guide holder having a body for securing an elongate medical wire or tube, such as a wire guide or catheter. The body is adapted to be attached to a scope or a bite block. The body can be provided with protrusions and/or grooves for holding a wire guide. The wire guide holder may be affixed to the medical scope by clamping. The wire guide holder can also be provided with a seal.

### BRIEF SUMMARY OF THE INVENTION

The present invention is defined in the appended claims. A seal device for use with an endoscope having a working channel extending from a working channel port is provided. The device includes a sleeve having a proximal portion, a distal portion, and a lumen extending therethrough. The proximal portion comprises an attachment mechanism that is adapted to attach to the working channel port of the endoscope, and the distal portion is adapted to conform around one or more medical devices that are inserted therethrough when subjected to a sufficient back pressure created by the proximal movement of fluids through the working channel. The one or more medical devices have an irregular cross sectional profile. The sleeve is configured for insertion into the working channel port of an endoscope.

Further, a seal device for use with an endoscope having a working channel extending from a working channel port is provided. The device includes a proximal portion, a distal portion, and a lumen extending therethrough. A diameter of the proximal portion is greater than a diameter of the distal portion. The sleeve is adapted to substantially seal around at least two medical devices inserted therethrough when a back pressure of about 20 mmHg exists within the working channel. The at least two medical devices have different diameters, and the at least two medical devices have a combined irregular cross-sectional profile. The sleeve creates minimal frictional force around the at least two medical devices inserted therethrough so as to not inhibit the movement thereof. The device, further includes an attachment mechanism adapted to attach the sleeve to the working channel port of the endoscope.

Also provided is a method for sealing a working channel of an endoscope that extends from a working channel port of the endoscope. The method includes providing a seal sleeve wherein the seal sleeve is adapted to substantially seal around one or more medical devices inserted therethrough when a sufficient back pressure is present, wherein the one or more medical devices have an irregular cross-sectional profile. The method further includes attaching the seal sleeve to the working channel port of the endoscope with an attachment mechanism. The method further includes introducing the one or more medical devices through the seal sleeve, and providing a back pressure sufficient to cause the seal sleeve to conform to the one or more medical devices.

Still further, a seal device for use with an endoscope having a working channel extending from a working channel port is provided. The seal device includes a sleeve having a proximal portion, a distal portion, and a lumen extending therethrough. The proximal portion includes an attachment mechanism that is adapted to attach to the working channel port of the endoscope, and the distal portion is adapted to conform around one or more medical devices that are inserted therethrough when subjected to a sufficient back pressure or a surface tension created by the proximal movement of fluids through the working channel. The one or more medical devices have an irregular cross sectional profile. The sleeve is configured for insertion into the working channel port of an endoscope.

Still further, a seal device for use with an endoscope having a working channel extending from a working channel port is provided. The seal device includes a sleeve having a proximal portion, a distal portion, and a lumen extending therethrough. A diameter of the proximal portion is greater than a diameter of the distal portion. The sleeve is adapted to substantially seal around at least two medical devices inserted therethrough when a back pressure of about 20 mmHg or a surface tension exists within the working channel. The at least two medical devices have different diameters. The at least two medical devices have a combined irregular cross-sectional profile. The sleeve creates minimal frictional force around the at least two medical devices inserted therethrough so as to not inhibit the movement thereof. The device further includes an attachment mechanism adapted to attach the sleeve to the working channel port of the endoscope.

Still further, a method for sealing a working channel of an endoscope that extends from a working channel port of the endoscope is provided. The method includes providing a seal sleeve wherein the seal sleeve is adapted to substantially seal around one or more medical devices inserted therethrough when a sufficient back pressure or a surface tension is present, wherein the one or more medical devices have an irregular cross-sectional profile, attaching the seal sleeve to the working channel port of the endoscope with an attachment mechanism, introducing the one or more medical devices through the seal sleeve, and providing a back pressure or a surface tension sufficient to cause the seal sleeve to conform to the one or more medical devices.

### BRIEF DESCRIPTION OF THE SEVERAL VIEWS OF THE DRAWINGS

The embodiments will be further described in connection with the attached drawing figures. Throughout the specification, like reference numerals and letters refer to like elements. It is intended that the drawings included as a part of this specification be illustrative of the embodiments and should in no way be considered as a limitation on the scope of the invention.
**Fig. 1** is a cross sectional view of a working channel of an endoscope having an embodiment depicted in an un-sealing state;
**Fig. 2** is a cross sectional view of a working channel of an endoscope having the same embodiment as that depicted in **Fig. 1** but here depicted in a sealing state;
**Fig. 3** is a partial cross sectional view of a working channel of an endoscope having an embodiment of a seal sleeve and an attachment mechanism for attaching the seal sleeve to the working channel of the endoscope;
**Fig. 4** is a partial cross sectional view of a working channel of an endoscope having an alternate embodiment of a seal sleeve and an attachment mechanism for attaching the seal sleeve to the working channel of the endoscope;
**Fig. 5** is a perspective view of a working channel of an endoscope having an alternate embodiment of a seal sleeve and an attachment mechanism for attaching the seal sleeve to the working channel of the endoscope;
**Fig. 6** is a perspective view of the circled portion 6 depicted in **Fig. 5****;**
**Fig. 7** is another perspective view of a working channel of an endoscope having an alternate embodiment of a seal sleeve and a mechanism for attaching the seal sleeve to the working channel of the endoscope;
**Fig. 8** is a perspective view of an alternate embodiment of a seal sleeve and an attachment mechanism for attaching the seal sleeve to the working channel of the endoscope; and
**Fig. 9** is a cross sectional view of a working channel of an endoscope having the same embodiment of a seal sleeve as that depicted in **Fig. 8****.**

### DETAILED DESCRIPTION OF PRESENTLY PREFERRED EMBODIMENTS

The embodiments provide an apparatus that is able to maintain a seal around one or more medical devices having a combined irregular cross-sectional profile that are simultaneously inserted through the working channel of the endoscope while at the same time creating minimal or no frictional force.

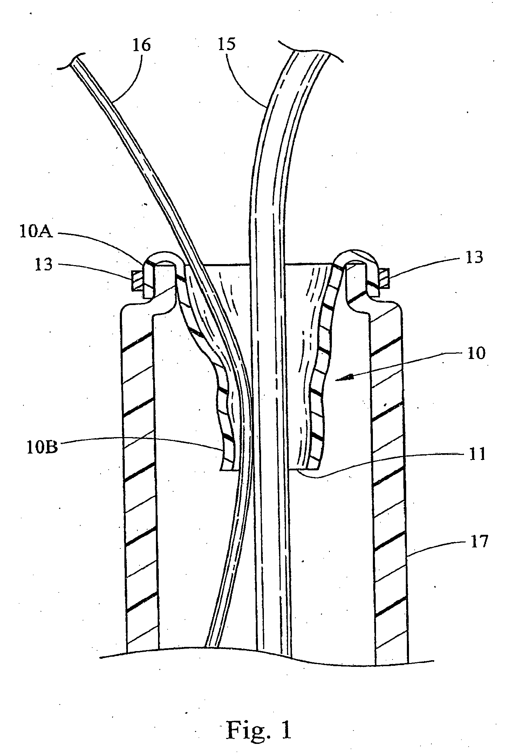

A more detailed description of the embodiments will now be given with reference to **Figs. 1-9****.** The present invention is not limited to those embodiments illustrated; it specifically contemplates other embodiments not illustrated but intended to be included in the claims.

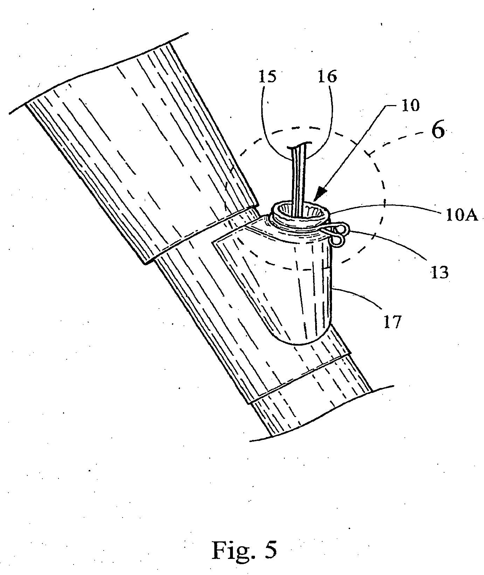

**Fig. 1** is a cross sectional view of a working channel **17** of an endoscope containing seal sleeve **10** in an un-sealing state. Seal sleeve has a proximal portion **10A** attached to the proximal end of working channel **17** via clamp **13.** Clamp **13** is further depicted in **Figs. 5****,** **6,** and **7****.** Distal portion **10B** of seal sleeve **10** is generally not fixedly attached to working channel **17.**

Seal sleeve **10** is made from a highly flexible, readily collapsible, substantially fluid-impermeable material having a low coefficient of friction, that will not degrade while in the presence of fluids within which it may come in contact. A preferred material is medical grade polyethylene; however seal sleeve **10** may be made from other materials that are flexible, readily collapsible, substantially fluid-impermeable, and that have a low coefficient of friction, including but not limited to, polyurethane, silicone, nylon, polyamides such as urethanes, polypropylene, polytetrafluoroethylene (PTFE), expanded polytetrafluoroethylene (ePTFE), and natural (latex) and polyisoprene rubbers. It is also contemplated that seal sleeve could be made from an elastic material that stretches when at least one medical device is placed therethrough, but then compresses and conforms around the medical device forming a substantially fluid-impermeable seal. Additionally, a removable stiffening insert may be added to seal sleeve **10** for easier placement of seal sleeve **10** within working channel **17.**

The thickness of seal sleeve **10** is about 0.001 - 0.025 inches, although greater and lesser thicknesses are also contemplated depending on the type of material from which seal sleeve **10** is made. The material and thickness of the material from which seal sleeve **10** is made should be such that it is able to conform around one or more medical devices, having a combined irregular cross-sectional profile, inserted therethrough, without becoming permanently deformed or stretched.

A substantially fluid-impermeable seal is formed when a sufficient back pressure or surface tension is present. The back pressure or surface tension causes seal sleeve **10** to collapse and fold back onto itself and conform around the medical devices inserted therethrough. Additionally, the material should be sufficiently stiff so that seal sleeve **10** does not entirely invert into itself. Furthermore, the frictional force exerted by seal sleeve **10** onto a medical instrument inserted therethrough should be low enough so that tactile feedback at proximal portion **10B** of seal sleeve **10** is preserved to allow the medical professional to obtain information from the distal end of the medical device inserted therethrough.

Wire guide **16** and catheter **15,** collectively having an irregular cross-sectional profile, enter through proximal portion **10A** of seal sleeve and exit through exit way **11** located at distal portion **10B** of seal sleeve **10.** Exit way **11** is large enough to allow medical devices to pass therethrough, but sufficiently small so as to be able to form a seal around each of the devices. Ideally, the diameter of exit way **11** is from about 0.020 - 0.200 inches, although other diameters are contemplated depending on the dimensions of the medical instrument inserted therethrough. Here, the inner diameter of exit way **11** is slightly larger than the combined outer diameters of the devices that will be placed through seal sleeve **10.** Proximal portion **10A** of seal sleeve is approximately about 0.157 - 0.197 inches wide, distal portion is approximately about 0.079 - 0.118 inches wide and seal sleeve **10** is approximately about 1 inch long; however, other dimensions are contemplated. Seal sleeve **10** is able to be configured so as to maintain a seal around one or more devices having various shapes and sizes that are inserted through working channel **17.** Seal sleeve **10** is not limited to having a cone-shape; other shapes include but are not limited to a cylinder and duck-bill shape.

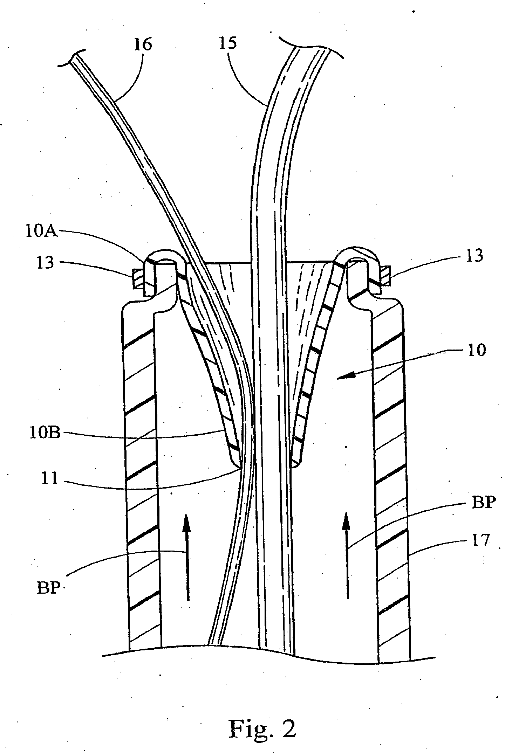

**Fig. 2** depicts the embodiment of **Fig. 1** but in a collapsed sealing state. Back pressure **BP** can occur naturally or as a result from insufflating an orifice. However, the embodiments disclosed herein do not require back pressure as the embodiments will also prevent leakage due to surface tension. Back pressure **BP** is generally up to about 20 mmHg and is such that it presses back up through working channel **17** in the proximal direction. Because seal sleeve **10** is made from a highly flexible, readily collapsible material, it will collapse onto itself in the proximal direction and conform around wire guide **16** and catheter **15** to form a substantially fluid-impermeable seal.

Because seal sleeve **10** is made from a material having a low coefficient of friction, the medical professional is able to use tactile feedback as a means for determining how wire guide **16** and catheter **15** ought to be further manipulated. Because a seal is maintained, the orifice remains insufflated and the area surrounding the working channel will not be contaminated by fluid escape.

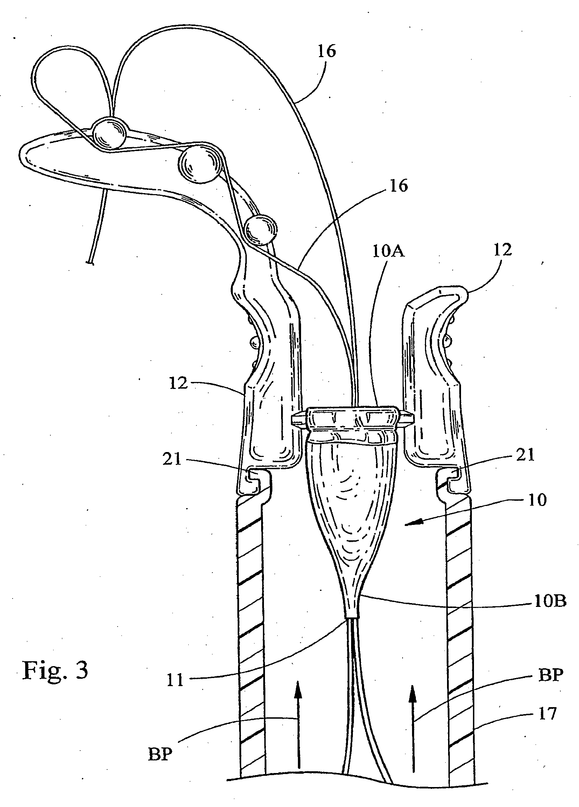

**Fig.** 3 depicts an embodiment of a seal sleeve **10** that is integrated into a Wire Guide Locking Device attachment mechanism, of the type produced by Cook Endoscopy. A wire guide locking device **12** is further described in U.S. Patent Application Serial No. 11/350,483. Seal sleeve **10** is attached to modified wire guide locking device **12** using a medically acceptable adhesive, however, other attachment mechanisms are contemplated. Wire guide locking device **12** attaches to working channel **17** having an outwardly extending flange **21.**

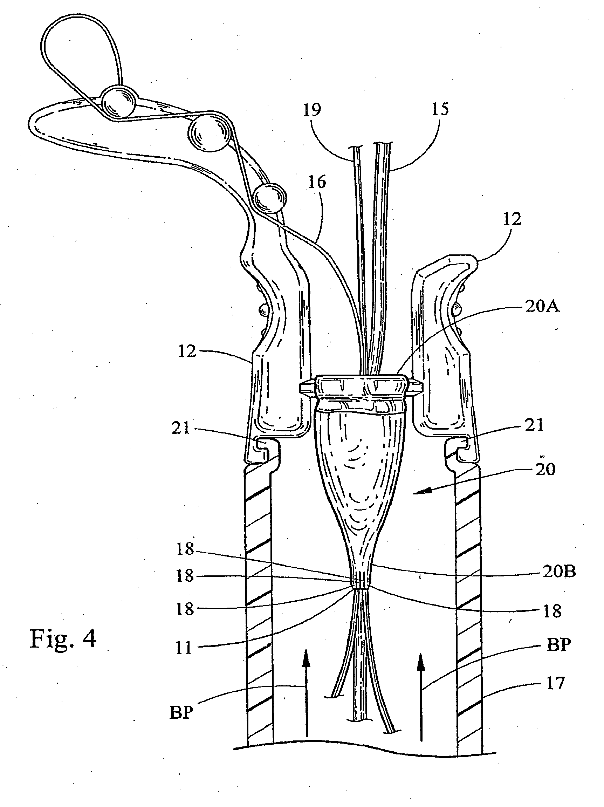

**Fig. 4** depicts an alternate embodiment of seal sleeve **20,** the proximal portion 20A of which is integrated into a modified wire guide locking device **12** as depicted in **Fig. 3****.** Distal portion **20B** of seal sleeve **20** includes a plurality of flaps **18.** Flaps **18** help to create a seal around a plurality of medical devices **15, 16,19,** having a combined irregular cross-sectional profile, inserted through seal sleeve **20.** In particular, when a sufficient back pressure or surface tension is present, a substantially fluid-impermeable seal is formed when flaps **18** fold onto themselves and conform around gaps that exist between the plurality of medical devices.

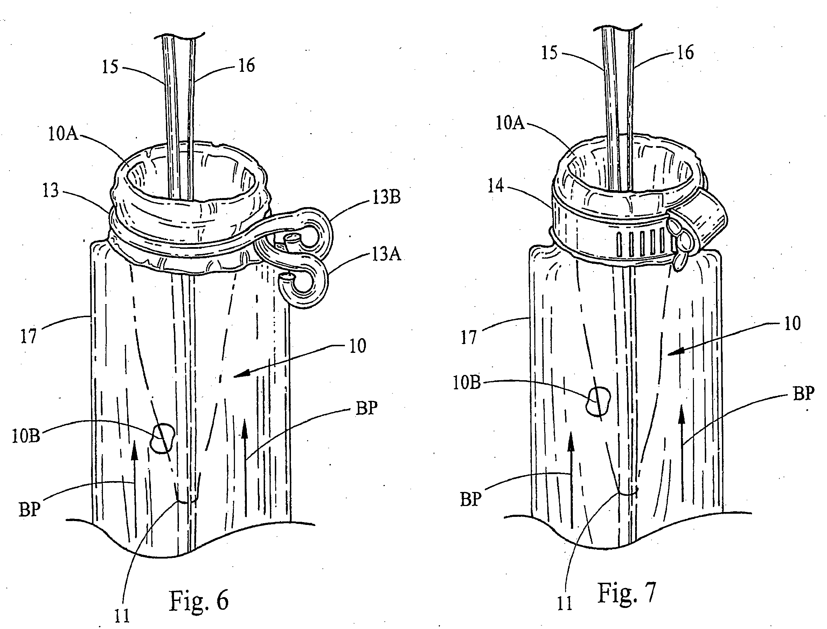

**Fig. 5** depicts an embodiment of a seal sleeve **10** and a mechanism for attaching seal sleeve **10** to working channel 17 of endoscope, and **Fig. 6** is a close-up view of the circled portion 6 depicted in **Fig. 5****.** Seal sleeve **10** is placed into working channel **17** such that proximal portion **10A** of seal sleeve **10** overlaps the end of working channel **17.** Clamp arms **13A** and **13B** of clamp **13** are pressed towards each other causing the diameter of clamp **13** to expand. Clamp **13** is then placed around proximal portion **10A** of seal sleeve **10** and clamp arms **13A** and **13B** are released causing clamp **13** to tighten and hold seal sleeve **10** firmly in place.

**Fig. 7** depicts an alternate mechanism for attaching seal sleeve **10** to working channel **17** of endoscope. Here, seal sleeve **10** is placed into working channel **17** such that proximal portion **10A** of seal sleeve **10** overlaps the end of working channel **17.** Clamp **14** is placed around proximal portion **10A** of seal sleeve **10** and is tightened so as to hold seal sleeve **10** firmly in place. Other attachment mechanisms are contemplated, including but not limited to, attaching seal sleeve **10** to working channel **17** using a medically acceptable adhesive, elastic, tape, or a cap.

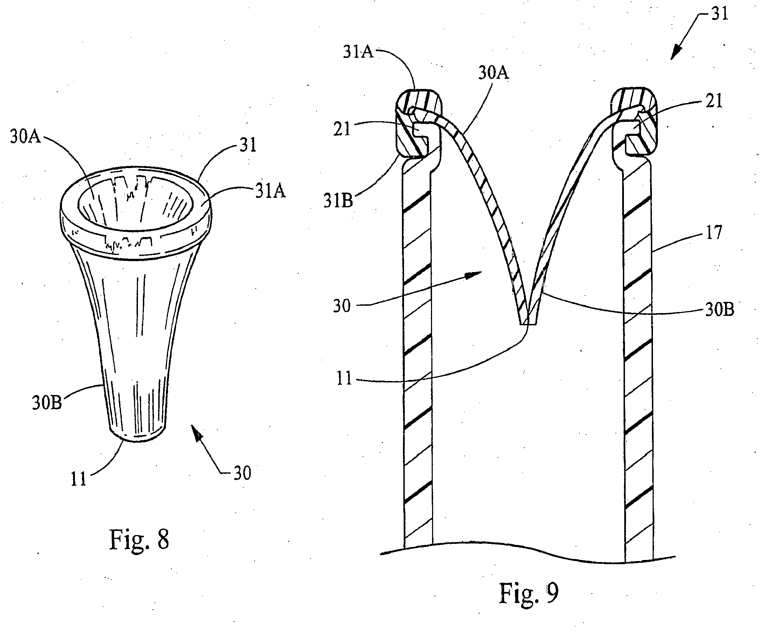

**Fig. 8** depicts a perspective view of an alternate embodiment of seal sleeve **30;** **Fig. 9** depicts a cross sectional view of seal sleeve **30** disposed within working channel **17.** Distal portion **30B** of seal sleeve **30** comprises exit way **11.** Proximal portion **30A** of seal sleeve **30** is fixedly compressed between cap **31** that comprises two pieces **31A, 31B.** Cap **31** removably attaches to outwardly extending flange **21** of working channel **17**. It is also contemplated that cap **31** could be threaded to attach to a working channel having treads. Cap **31** could also be lined with a non-permanent adhesive substance so that cap **31** remains attached to a working channel lacking outwardly extending flange **21.** Cap **31** can also be lined with a tacky material or further comprise a gasket to aid in attaching to a working channel lacking outwardly extending flange **21.** Additionally, cap **31** can be configured in such a way that it snaps over a working channel lacking outwardly extending flange **21.**

As is evident, the embodiments provide a very effective solution for maintaining a seal around one or more devices having an irregular cross-sectional profile that are simultaneously inserted into the working channel of an endoscope. The foregoing description and drawings are provided for illustrative purposes only and are not intended to limit the scope of the invention described herein or with regard to the details of its construction and manner of operation. It will be evident to one skilled in the art that modifications and variations may be made without departing from the spirit and scope of the invention. Changes in form and in the proportion of parts, as well as the substitution of equivalents, are contemplated as circumstances may suggest and render expedience; although specific terms have been employed, they are intended in a generic and descriptive sense only and not for the purpose of limiting the scope of the invention set forth in the following claims.

## Claims

1. An assembly comprising a seal device and an endoscope having a working channel (17) extending from a working channel port, the seal device comprising:
a sleeve (10) having a proximal portion (10a), a distal portion (10b), and a lumen extending therethrough, the sleeve being inserted into the working channel port of the endoscope;
**characterized in that** the proximal portion comprises an attachment mechanism (13, 14, 31) that is attached to the working channel port of the endoscope, and the distal portion is inserted into the working channel port and is adapted to conform around at least two medical devices (15,16) that are inserted therethrough when subjected to a back pressure of about 20 mmHg or a surface tension created by the proximal movement of fluids through the working channel; and
wherein the at least two medical devices have a combined irregular cross sectional profile.

2. The assembly of Claim 1 wherein the sleeve is made from a material selected from the group consisting of polyethylene, polyurethane, silicone, nylon, polyamides, urethanes, polypropylene, polytetrafluoroethylene, expanded polytetrafluoroethylene, latex, polyisoprene rubbers, and an elastic material.

3. The assembly of Claim 1 wherein the sleeve is made from a material that is 0.025 - 0.63mm (0.001-0.025 inches) thick.

4. The assembly of Claim 1 wherein the distal portion further comprises a plurality of flaps.

5. The assembly of Claim 1 wherein the sleeve shape is selected from the group consisting of a cone, cylindrical, or duck-bill.

6. The assembly of Claim 1 wherein the at least two medical devices comprise a wire guide and a catheter.

7. The assembly of Claim 1 wherein the at least two medical devices inserted therethrough have different diameters.

8. The assembly of Claim 1 wherein the sleeve creates minimal frictional-force around the at least two medical devices inserted therethrough so as to not inhibit the movement thereof.

9. The assembly of Claim 1 wherein the attachment mechanism is selected from the group consisting of a clamp, wire guide locking device, tape, elastic, adhesive, and a cap.

10. A method for sealing a working channel of an endoscope, the method comprising:
providing an endoscope having a working channel that extends from a working channel port of the endoscope;
**characterized by** providing a seal sleeve wherein the seal sleeve is adapted to seal around at least two medical devices inserted therethrough when a sufficient back pressure or a surface tension is present, wherein the at least two medical devices have a combined irregular cross-sectional profile,
attaching the seal sleeve to the working channel port of the endoscope with an attachment mechanism; inserting the distal portion of the sleeve into the working channel; introducing the at least two medical devices through the seal sleeve;
and
providing a back pressure or a surface tension sufficient to cause the seal sleeve to conform to the at least two medical devices.

11. The method of Claim 10 wherein the back pressure introduced is about 20mmHg.

12. The method of Claim 10 wherein the attachment mechanism is selected from the group consisting of a clamp, wire guide locking device, tape, elastic, adhesive, and a cap.

13. The method of Claim 10 wherein the at least two medical devices introduced comprise a wire guide and a catheter.

14. The method of Claim 10 wherein the at least two medical devices have different diameters.

15. The method of Claim 10 wherein the seal sleeve provided creates minimal frictional force around the at least two medical devices introduced so as to not inhibit the movement thereof.

## Patentansprüche

1. Anordnung, umfassend eine Dichtungsvorrichtung und ein Endoskop mit einem Arbeitskanal (17), der sich von einem Arbeitskanalanschluss erstreckt, wobei die Dichtungsvorrichtung
eine Hülse (10) mit einem proximalen Abschnitt (10a), einem distalen Abschnitt (10b) und einem sich dort hindurch erstreckenden Lumen umfasst, wobei die Hülse in den Arbeitskanalanschluss des Endoskops eingeführt ist,
**dadurch gekennzeichnet, dass** der proximale Abschnitt einen Anbringmechanismus (13, 14, 31) umfasst, der am Arbeitskanalanschluss des Endoskops angebracht ist, und der distale Abschnitt in den Arbeitskanalanschluss eingeführt und geeignet ist, sich um mindestens zwei dort hindurch eingeführte medizinische Vorrichtungen (15, 16) herum anzupassen, wenn er mit einem Gegendruck von ungefähr 20 mmHg oder einer durch die proximale Bewegung von Fluiden durch den Arbeitskanal erzeugten Oberflächenspannung beaufschlagt ist, und
wobei die mindestens zwei medizinischen Vorrichtungen ein kombiniertes unregelmäßiges Querschnittsprofil haben.

2. Anordnung nach Anspruch 1, wobei die Hülse aus einem Material hergestellt ist, das aus der aus Polyethylen, Polyurethan, Silikon, Nylon, Polyamiden, Urethanen, Polypropylen, Polytetrafluorethylen, expandiertem Polytetrafluorethylen, Latex, Polyisopren-Kautschuken und einem elastischen Material bestehenden Gruppe ausgewählt ist.

3. Anordnung nach Anspruch 1, wobei die Hülse aus einem Material hergestellt ist, das 0,025 - 0,63 mm (0,001 - 0,025 Zoll) dick ist.

4. Anordnung nach Anspruch 1, wobei der distale Abschnitt ferner eine Vielzahl von Klappen umfasst.

5. Anordnung nach Anspruch 1, wobei die Hülsenform aus der aus einem Kegel, einem Zylinder oder einem Entenschnabel bestehenden Gruppe ausgewählt ist.

6. Anordnung nach Anspruch 1, wobei die mindestens zwei medizinischen Vorrichtungen eine Drahtführung und einen Katheter umfassen.

7. Anordnung nach Anspruch 1, wobei die mindestens zwei dort hindurch eingeführten medizinischen Vorrichtungen unterschiedliche Durchmesser haben.

8. Anordnung nach Anspruch 1, wobei die Hülse um die mindestens zwei dort hindurch eingeführten medizinischen Vorrichtungen eine minimale Reibungskraft erzeugt, um deren Bewegung nicht zu behindern.

9. Anordnung nach Anspruch 1, wobei der Anbringmechanismus aus der aus einer Klemme, einer Drahtführungsarretierungsvorrichtung, einem Band, einem Gummiband, einem Klebstoff und einer Kappe bestehenden Gruppe ausgewählt ist.

10. Verfahren zum Abdichten eines Arbeitskanals eines Endoskops, umfassend:
Bereitstellen eines Endoskops mit einem Arbeitskanal, der sich von einem Arbeitskanalanschluss des Endoskops erstreckt,
**gekennzeichnet durch** Bereitstellen einer Dichtungshülse, wobei die Dichtungshülse geeignet ist,
um mindestens zwei dort hindurch eingeführte medizinische Vorrichtungen herum abzudichten, wenn ein hinreichender Gegendruck oder eine Oberflächenspannung vorliegt, wobei die mindestens zwei medizinischen Vorrichtungen ein kombiniertes unregelmäßiges Querschnittsprofil haben,
Anbringen der Dichtungshülse am Arbeitskanalanschluss des Endoskops mit einem Anbringmechanismus,
Einführen des distalen Abschnitts der Hülse in den Arbeitskanal,
Einführen der mindestens zwei medizinischen Vorrichtungen **durch** die Dichtungshülse
und
Bereitstellen eines ausreichenden Gegendrucks oder einer ausreichenden Oberflächenspannung, um zu veranlassen, dass sich die Dichtungshülse an die mindestens zwei medizinischen Vorrichtungen anpasst.

11. Verfahren nach Anspruch 10, wobei der eingeführte Gegendruck ungefähr 20 mmHg beträgt.

12. Verfahren nach Anspruch 10, wobei der Anbringmechanismus aus der aus einer Klemme, einer Drahtführungsarretierungsvorrichtung, einem Band, einem Gummiband, einem Klebstoff und einer Kappe bestehenden Gruppe ausgewählt ist.

13. Verfahren nach Anspruch 10, wobei die mindestens zwei eingeführten medizinischen Vorrichtungen eine Drahtführung und einen Katheter umfassen.

14. Verfahren nach Anspruch 10, wobei die mindestens zwei medizinischen Vorrichtungen unterschiedliche Durchmesser haben.

15. Verfahren nach Anspruch 10, wobei die bereitgestellte Dichtungshülse um die mindestens zwei eingeführten medizinischen Vorrichtungen eine minimale Reibungskraft erzeugt, um deren Bewegung nicht zu behindern.

## Revendications

1. Ensemble comportant un dispositif d'étanchéité et un endoscope présentant un canal (17) de travail qui s'étend depuis un orifice de canal de travail, le dispositif d'étanchéité comportant :
une manche (10) présentant une partie (10a) proximale, une partie (10b) distale, et une lumière traversante, la manche étant insérée dans l'orifice de canal de travail de l'endoscope ;
**caractérisé en ce que** la partie proximale comporte un mécanisme (13, 14, 31) de fixation qui est fixé sur l'orifice de canal de travail de l'endoscope, et la partie distale est insérée dans l'orifice de canal de travail et est apte à se conformer autour d'au moins deux dispositifs (15, 16) médicaux qui sont insérés dans celle-ci lorsqu'ils sont soumis à une pression de retour d'environ 20 mmHg ou une tension superficielle créée par le déplacement proximal des fluides dans le canal de travail ; et
dans lequel lesdits au moins deux dispositifs médicaux ont un profil combiné irrégulier en coupe.

2. Ensemble selon la revendication 1 dans lequel la manche est fabriquée à partir d'une matière sélectionnée dans le groupe constitué par le polyéthylène, le polyuréthane, la silicone, le nylon, les polyamides, les uréthanes, le polypropylène, le polytétrafluoroéthylène, le polytétrafluoroéthylène expansé, le latex, les caoutchoucs de polyisoprène, et une matière élastique.

3. Ensemble selon la revendication 1 dans lequel la manche est fabriquée à partir d'une matière d'une épaisseur de l'ordre de 0,025 à 0,63 mm (0,001 à 0,025 pouce).

4. Ensemble selon la revendication 1 dans lequel la partie distale comporte en outre une pluralité de rabats.

5. Ensemble selon la revendication 1 dans lequel la forme de la manche est sélectionnée dans le groupe constitué par un cône, un cylindre, ou un bec de canard.

6. Ensemble selon la revendication 1 dans lequel lesdits au moins deux dispositifs médicaux comportent un fil de guidage et un cathéter.

7. Ensemble selon la revendication 1 dans lequel lesdits au moins deux dispositifs médicaux insérés dans la manche ont des diamètres différents.

8. Ensemble selon la revendication 1 dans lequel la manche crée une force de frottement minimale autour desdits au moins deux dispositifs médicaux insérés dans celle-ci de façon à ne pas empêcher leur déplacement.

9. Ensemble selon la revendication 1 dans lequel le mécanisme de fixation est sélectionné dans le groupe constitué par une pince, un dispositif de verrouillage de fil de guidage, une bande, un élastique, un adhésif, et un capuchon.

10. Procédé destiné à assurer l'étanchéité d'un canal de travail d'un endoscope, le procédé consistant à :
fournir un endoscope présentant un canal de travail qui s'étend depuis un orifice de travail de canal de l'endoscope ;
**caractérisé par** la fourniture d'une manche d'étanchéité dans lequel la manche d'étanchéité est apte à assurer l'étanchéité autour desdits au moins deux dispositifs médicaux insérés dans celle-ci lorsqu'une pression de retour suffisante ou une tension superficielle est présente, lesdits au moins deux dispositifs médicaux ayant un profil combiné irrégulier en coupe,
fixer la manche d'étanchéité sur l'orifice de canal de travail de l'endoscope au moyen d'un mécanisme de fixation ;
insérer la partie distale de la manche dans le canal de travail ;
introduire lesdits au moins deux dispositifs médicaux dans la manche d'étanchéité ; et
fournir une pression de retour ou une tension superficielle suffisante pour amener la manche d'étanchéité à se conformer auxdits au moins deux dispositifs médicaux.

11. Procédé selon la revendication 10 dans lequel la pression de retour introduite est d'environ 20 mmHg.

12. Procédé selon la revendication 10 dans lequel le mécanisme de fixation est sélectionné dans le groupe constitué par une pince, un dispositif de verrouillage de fil de guidage, une bande, un élastique, un adhésif, et un capuchon.

13. Procédé selon la revendication 10 dans lequel lesdits au moins deux dispositifs médicaux introduits comportent un fil de guidage et un cathéter.

14. Procédé selon la revendication 10 dans lequel lesdits au moins deux dispositifs médicaux ont des diamètres différents.

15. Procédé selon la revendication 10 dans lequel la manche d'étanchéité fournie crée une force de frottement minimale autour desdits au moins deux dispositifs médicaux introduits de façon à ne pas empêcher leur mouvement.
